(19) Europäisches Patentamt
European Patent Office
Office européen des brevets

(11) Publication number: **0 397 418 A1**

(12) **EUROPEAN PATENT APPLICATION**

(21) Application number: **90304881.7**

(51) Int. Cl.[5]: **C07C 69/76, C07C 67/36**

(22) Date of filing: **04.05.90**

(30) Priority: **10.05.89 JP 116767/89**

(43) Date of publication of application: **14.11.90 Bulletin 90/46**

(84) Designated Contracting States: **DE FR GB**

(71) Applicant: **Director General, Agency of Industrial Science and Technology**
**1-3-1, Kasumigaseki Chiyoda-ku**
**Tokyo 100(JP)**

Applicant: **PETROLEUM ENERGY CENTER**
**2-3-22, Azabudai**
**Minato-ku, Tokyo(JP)**

(72) Inventor: **Sugi, Yoshihiro**
**7-222-B504 Shin-Matsudo**
**Matsudo-Shi, Chiba-Ken(JP)**
Inventor: **Iwai, Yoshikazu**
**20-7 Isogodai, Isogo-Ku**
**Yokohama-Shi, Kanagawa-Ken(JP)**
Inventor: **Takeuchi, Kazuhiko**
**805-202 Azuma 2-chome**
**Tsukuba-Shi, Ibaraki-Ken(JP)**
Inventor: **Takagi, Satoru**
**241 Suenaga, Takatsu-Ku**
**Kawasaki-Shi, Kanagawa-Ken(JP)**
Inventor: **Doi, Yoshiaki**
**21-14 Shiratoridai, Midori-ku**
**Yokohama-Shi, Kanagawa-Ken(JP)**

(74) Representative: **Horton, Sophie Emma et al**
**Elkington and Fife Beacon House 113 Kingsway**
**London WC2B 6PP(GB)**

(54) **9,10-dihydrophenanthrene-2,7-dicarboxylic acid diesters, 7-Bromo-9,10-dihydrophenanthrene-2-carboxylic acid esters and a process for producing the same.**

(57) A novel 9,10-dihydrophenanthrene2,7-dicarboxylic acid diester represented by the general formula (I):

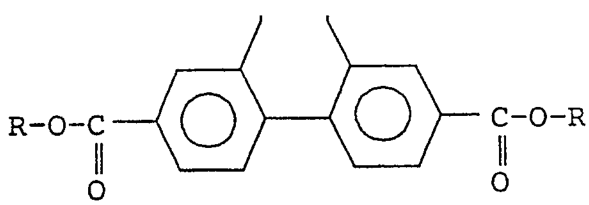

and a novel 7-bromo-9,10-dihydrophenanthrene-2-carboxylic acid monoester represented by the general formula (II):

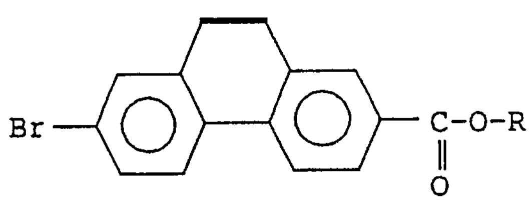

wherein each R represents a hydrocarbon group containing from 1 to 20 carbon atoms. A process for selectively synthesizing the compound of the general formula (I) and the compound of the general formula

EP 0 397 418 A1

(II) comprising reacting 2,7-dibromo-9,10-dihydrophenanthrene with carbon monoxide and an alcohol of the general formula (III) R-OH in the presence of a base and a palladium-phosphine catalyst. Such novel compounds of the general formula (I) and (II) can be produced in a high yield and selectivity from inexpensive raw materials by simple reaction steps.

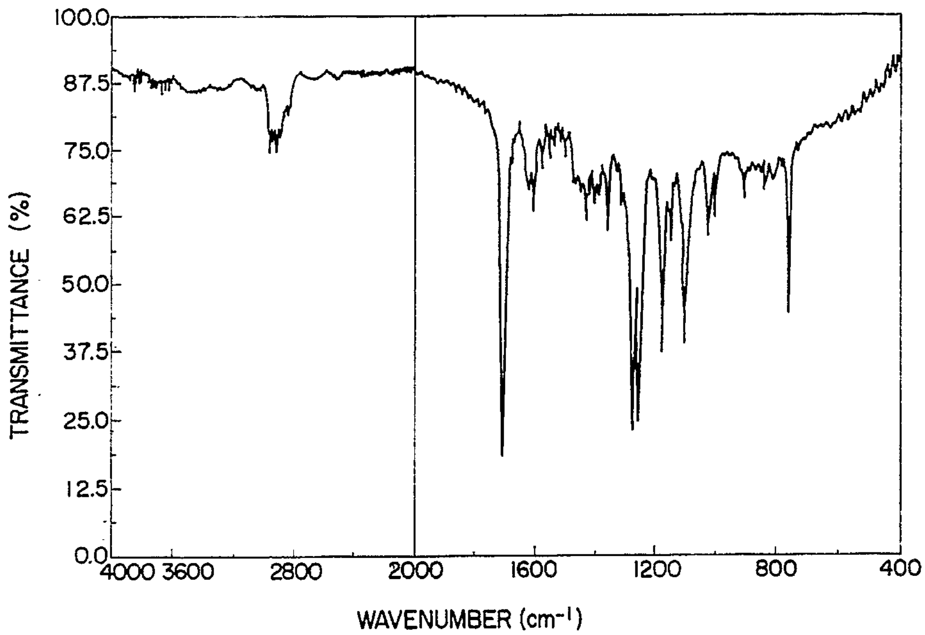


FIG. 1

# 9,10-DIHYDROPHENANTHRENE-2,7-DICARBOXYLIC ACID DIESTERS, 7-BROMO-9,10-DIHYD ROPHENANTHRENE-2-CARBOXYLIC ACID ESTERS AND A PROCESS FOR PRODUCING THE SAME

## BACKGROUND OF THE INVENTION

This invention relates to novel 9,10-dihydrophenanthrene-2,7-dicarboxylic acid diesters and 7-bromo-9,10-dihydrophenanthrene-2-carboxylic acid esters and a process for producing the same. More particularly, it relates to a process for producing 9,10-dihydrophenanthrene-2,7-dicarboxylic acid diesters or 7-bromo-9,10-dihydrophenanthrene-2-carboxylic acid esters wherein 2,7-dibromo-9,10-dihydrophenanthrene is reacted with carbon monoxide and an alcohol in the presence of a base and a palladium-phosphine catalyst.

Heretofore, there have been proposed various processes for carrying out the carbonylation reaction of aromatic compounds. Examples of such processes include a process for producing a diphenyl carboxylic acid wherein an iodinated or brominated diphenyl is reacted with carbon monoxide in an aqueous solvent in the presence of a catalyst (Japanese Patent Laid-Open Publication No. 185055/1987); a process for producing a hydroxy aromatic carboxylic ester wherein a halogenated aromatic hydroxy compound is reacted with carbon monoxide in an alcohol solvent in the presence of a catalyst (Japanese Patent Laid-Open Publication No. 187435/1987); a process for producing an aromatic carboxylic acid wherein an aromatic iodine compound is reacted with carbon monoxide in the presence of a base and water without using any catalyst (Japanese Patent Laid-Open Publication No. 5052/1988); and a process for producing an; aromatic carboxylic acid ester wherein a halogenated aromatic compound is reacted with carbon monoxide at a high temperature under a high pressure in the presence of a catalyst (U.S. Patent No. 3,636,082).

We have carried out studies with respect to carbonylation reaction of aromatic compounds. In particular, we have carried out extensive reviews with respect to effects of raw materials, catalysts and reaction conditions on the yield and selectivity of products. We have reviewed the carbonylation reaction of 2,7-dibromodihydrophenanthrene in detail. We have now found that 9,10-dihydrophenanthrene-2,7-dicarboxylic acid diesters and 7-bromo-9,10-dihydrophenanthrene carboxylic acid esters which have never been known in the prior art processes described above can be synthesized in a good yield under specific conditions.

## SUMMARY OF THE INVENTION

Thus, the present invention provides a 9,10-dihydrophenanthrene-2,7-dicarboxylic acid diester represented by the general formula (I):

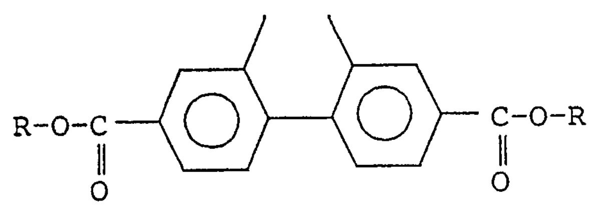

or a 7-bromo-9,10-dihydrophenanthrene-2-carboxylic acid ester represented by the general formula (II):

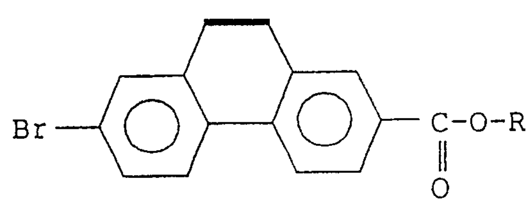

wherein each R represents a hydrocarbon group containing from 1 to 20 carbon atoms.

The present invention also provides a process for producing compounds of the general formulae (I) and (II) described above by reacting a 2,7-dibromo-9,10-dihydrophenanthrene with carbon monoxide and an alcohol of the general formula (III) R-OH wherein R is as defined above, in the presence of a base and a palladium-phosphine catalyst.

## BRIEF DESCRIPTION OF THE DRAWINGS

FIG. 1 is an IR spectrum of diethyl 9,10-dihydrophenanthrene-2,7-dicarboxylate obtained in Example 1 as a chief product;

FIG. 2 is a mass spectrum of diethyl 9,10-dihydrophenanthrene-2,7-dicarboxylate obtained in Example 1 as a chief product;

FIG. 3 is an IR spectrum of ethyl 7-bromo-9,10-dihydrophenanthrene-2-carboxylate obtained in Example 3 as a chief product; and

FIG. 4 is a mass spectrum of ethyl 7-bromo-9,10-dihydrophenanthrene-2-carboxylate obtained in Example 3 as a chief product.

## DETAILED DESCRIPTION OF THE INVENTION

The present invention will be described in detail.

The present invention relates to a 9,10-dihydrophenanthrene-2,7-dicarboxylic acid diester represented by the general formula (I):

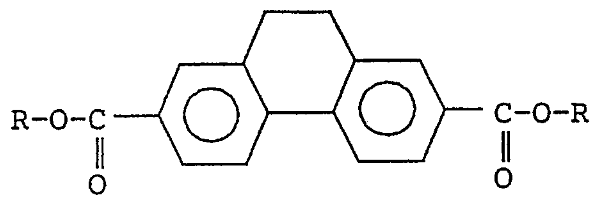

or a 7-bromo-9,10-dihydrophenanthrene-2-carboxylic acid ester represented by the general formula (II):

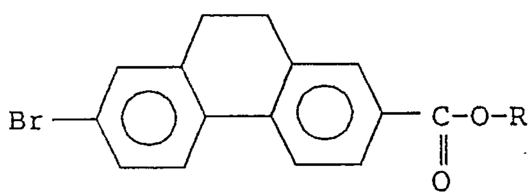

R in the formulae (I) and (II) described above is a hydrocarbon group containing from 1 to 20 carbon atoms. R represents an aliphatic, alicyclic or aromatic hydrocarbon group including alkyl groups such as methyl and ethyl; cycloalkyl groups such as cyclopentyl and cyclohexyl; and aryl groups such as benzyl and phenyl. Thus, representative examples of such novel compounds according to the present invention represented by the general formulae (I) and (II) include diethyl 9,10- dihydrophenanthrene-2,7-dicarboxylate and ethyl 7-bromo-9,10-dihydrophenanthrene-2-carboxylate.

These compounds can be utilized as raw materials for heat-resistant or liquid crystal polymers. Further, the compounds can be utilized as raw materials for various drugs and agricultural chemicals.

According to the present invention, such compounds can be produced by reacting a 2,7-dibromo-9,10-dihydrophenanthrene with carbon monoxide and an alcohol of the general formula (III) R-OH wherein R is as defined above in the presence of a base and a palladium-phosphine catalyst.

The 2,7-dibromo-9,10-dihydrophenanthrenes from which the desired product is produced can be readily obtained by various methods. For example, the 2,7-dibromo-9,10-dihydrophenanthrenes are obtained in a high yield by reacting 9,10-dihydrophenanthrene in a trimethylphosphate solvent in the presence of bromine (D.E. Pearson, U.S. Patent No. 3,988,369 (1976)).

The base used in the present invention has such an effect that it remarkably accelerates the reaction. The addition of such a base reduces the reaction time and reaction temperature. Both organic bases and inorganic bases can be used as the base. That is, any bases can be used so long as they can capture hydrogen bromide which forms in the course of the reaction. Examples of such bases include aliphatic amines, aromatic amines, quaternary ammonium hydroxide, alkali metal alcoholates, alkali metal hydroxides, alkali metal carbonates, alkaline earth metal alcoholates, alkaline earth metal hydroxides and alkaline earth metal carbonates. Of these, aliphatic amines such as triethylamine, ethyldiisopropylamine and butyldimethylamine are preferred. The amount of the base used is from 0.01 to 10 molar equivalents,

preferably from 0.3 to 5 molar equivalents based on the amount of bromine in the 2,7-dibromo-9,10-dihydrophenanthrene to be reacted.

The catalyst used in the present invention is a palladium-phosphine catalyst. Palladium compounds which are a source of palladium include palladium chloride and palladium sulfate. The phosphines include aromatic phosphines such as triphenylphosphine, tritolylphosphine and trianisylphosphine; alkylphosphines such as tributylphosphine, tripropylphosphine and triisopropylphosphine; and diphosphines such as 1,2-bisdiphenylphosphinomethane and 1,3-bisdiphenylphosphinopropane. Of these, a preferred palladium compound is palladium chloride, and preferred phosphines are aromatic phosphines such as triphenylphosphine. These phosphines may be added to a reaction system as a palladium complex. Alternatively, the phosphines may be charged into a reactor together with salts such as palladium chloride to form their complex in a system. The amount of the palladium compound used is from 1/10 to 1/1000 molar equivalent, preferably from 1/20 to 1/200 molar equivalent based on the 2,7-dibromo-9,10-dihydrophenanthrene to be reacted. The amount of the phosphine compound is from 0.5 to 30 molar equivalents, preferably from 1 to 25 molar equivalents based on palladium. When the amount of the phosphine compound added is small, a 7-bromo-9,10-dihydrophenanthrene-2-carboxylic acid ester is a chief product. When the amount of the phosphine compound added is large, a 9,10-dihydrophenanthrene-2,7-dicarboxylic acid diester is a chief product.

The alcohols used in the present invention are represented by the general formula (III) R-OH wherein R is as defined above. The alcohols used may vary depending upon purposes. Aliphatic alcohols, alicyclic alcohols and aromatic alcohols can be used herein. Preferred aliphatic alcohols are methanol, ethanol and propanol. Preferred alicyclic alcohols are cyclopentanol and cyclohexanol. Preferred aromatic alcohols are benzyl alcohol and phenol The amount of the alcohol used is at least one molar equivalent based on bromine in 2,7-dibromo-9,10-dihydrophenanthrene to be reacted.

Suitable reaction apparatuses are continuous or batch high-pressure reaction apparatuses conventionally used. The reaction apparatuses are not limited thereto. The reaction is carried out under a carbon monoxide pressure. The pressure of a carbon monoxide gas is a gauge pressure of from 5 to 200 $kg/cm^2$, preferably from 50 to 150 $kg/cm^2$. A considerably excess amount of carbon monoxide is generally used. The reaction temperature is from 50° to 200° C, preferably from 100° to 180° C from the standpoint of reaction rate. If the reaction temperature is above 200° C, side reaction may occur in a considerable extent.

While the novel compounds represented by the general formulae (I) and (II) which can be utilized as raw materials for liquid crystal polymers are obtained in this manner, various by-products tend to be simultaneously formed. Accordingly, various conditions can be adjusted in the present invention. The formation of various by-products is inhibited by adjusting various conditions when the amount of the base is relatively small, when the pressure of carbon monoxide is low or when the temperature is low, while other conditions are constant and thus the compounds described above can be in a high yield and selectivity as a chief product. However, when all of these conditions are satisfied, a reduction in reaction rate is caused, contrary to expectation. Therefore, it is necessary to make adjustments in order to prevent this reduction in reaction rate.

The following nonlimiting examples illustrate the present invention.

## EXAMPLE 1

An autoclave was charged with 8.45 grams (25 mmol) of 2,7-dibromo-9,10-dihydrophenanthrene, 6.07 grams (60 mmol) of triethylamine, 44.3 milligrams (0.25 mmol) of palladium chloride, 656 milligrams (2.5 mmol) of triphenylphosphine, 20 milliliters of ethanol and 20 milliliters of benzene. Carbon monoxide was then charged into the autoclave at a level of 50 $kg/cm^2$. The reaction mixture was heated to 120° C to carry out the reaction for 5 hours. After reaction, unreacted carbon monoxide was purged and the reaction solution was then withdrawn. The analysis was carried out by gas chromatography. As a result, 99.7% of the charged 2,7-dibromo-9,10-dihydrophenanthrene reacted. 7.84 grams (24.2 mmol) of diethyl 9,10-dihydrophenanthrene-2,7-dicarboxylate were obtained as a chief product (selectivity of 97%). (Melting point of 65.0° C - 67.0° C; elemental analysis C = 74.0%, H = 6.1%, 0 = 19.9%) The IR spectrum and mass spectrum of the product are shown in FIGS. 1 and 2, respectively. Besides, 82 milligrams (6 mmol) of ethyl 7-bromo-9,10-dihydrophenanthrene-2-carboxylate were obtained as a by-product (selectivity of 1%).

## EXAMPLE 2

Anautoclave was charged with 8.45 grams (25 mmol) of 2,7-dibromo-9,10-dihydrophenanthrene, 6.07 grams (60 mmol) of triethylamine, 44.3 milligrams (0.25 mmol) of palladium chloride, 262.4 milligrams (1 mmol) of triphenylphosphine, 20 milliliters of ethanol and 20 milliliters of benzene. Carbon monoxide was then charged into the autoclave at a level of 50 $kg/cm^2$. The reaction mixture was heated to 180 °C to carry out the reaction for 5 hours. After reaction, unreacted carbon monoxide was purged and the reaction solution was then withdrawn. The analysis was carried out by gas chromatography. As a result, 98.5% of the charged 2,7-dibromo-9,10-dihydrophenanthrene reacted. 7.22 grams (22.3 mmol) of diethyl 9,10-dihydrophenanthrene-2,7-dicarboxylate were obtained as a chief product (selectivity of 90.4%). Besides, 363 milligrams (1.1 mmol) of ethyl 7-bromo-9,10-dihydrophenanthrene-2-carboxylate were obtained as a by-product (selectivity of 4.5%).

## EXAMPLE 3

An autoclave was charged with 8.45 grams (25 mmol) of 2,7-dibromo-9,10-dihydrophenanthrene, 6.07 grams (60 mmol) of triethylamine, 44.3 milligrams (0.25 mmol) of palladium chloride, 65.6 milligrams (0.25 mmol) of triphenylphosphine, 20 milliliters of ethanol and 20 milliliters of benzene. Carbon monoxide was then charged into the autoclave at a level of 50 $kg/cm^2$. The reaction mixture was heated to 180 °C to carry out the reaction for 5 hours. After reaction, unreacted carbon monoxide was purged, and the reaction solution ,was withdrawn. The analysis was carried out by gas chromatography. As a result, 72% of the charged 2,7-dibromo-9,10-dihydrophenanthrene reacted. 3.64 grams (11 mmol) of ethyl 7-bromo-9,10-dihydrophenanthrene-2-carboxylate were obtained as a chief product (selectivity of 59%). (Melting point of 75.3 °C - 77.3 °C; elemental analysis C = 61.5%, H = 4.4%, 0 = 9.8%, Br = 24.3%) The IR spectrum and mass spectrum of the chief product are shown in FIGS. 3 and 4, respectively. Besides, 1.95 gram (6 mmol) of diethyl 9,10-dihydrophenanthrene-2,7-dicarboxylate was obtained as a by-product (selectivity of 33%).

## EXAMPLE 4

An autoclave was charged with 4.23 grams (12.5 mmol) of 2,7-dibromo-9,10-dihydrophenanthrene, 1.52 gram (15 mmol) of triethylamine, 22.2 milligrams (0.125 mmol) of palladium chloride, 65.6 milligrams (0.25 mmol) of triphenylphosphine, 10 milliliters of ethanol and 20 milliliters of benzene. The reaction was carried out for 3 hours at a temperature of 150 °C under a carbon monoxide pressure of 50 $kg/cm^2$ as in Example 1. As a result, 70% of the charged 2,7-dibromo-9,10-dihydrophenanthrene reacted. 1.85 gram (5.6 mmol) of ethyl 7-bromo-9,10-dihydrophenanthrene-2-carboxylate was obtained as a chief product (selectivity of 64%). Besides, 0.58 gram (1.8 mmol) of diethyl 9,10-dihydrophenanthrene-2,7- dicarboxylate was obtained as a by-product (selectivity of 20%).

## EXAMPLE 5

An autoclave was charged with 4.23 grams (12.5 mmol) of 2,7-dibromo-9,10-dihydrophenanthrene, 3.87 grams (30 mmol) of ethyldiisopropylamine, 22.2 milligrams (0.125 mmol) of palladium chloride, 65.6 milligrams (0.25 mmol) of triphenylphosphine, 10 milliliters of ethanol and 20 milliliters of benzene. The reaction was carried out for 3 hours at a temperature of 150 °C under a carbon monoxide pressure of 50 $kg/cm^2$ as in Example 1. As a result, 41% of the charged 2,7-dibromo-9,10-dihydrophenanthrene reacted. 1.39 gram (4.2 mmol) of ethyl 7-bromo-9,10-dihydrophenanthrene-2-carboxylate was obtained as a chief product (selectivity of 80.5%). Besides, 3 milligrams (0.01 mmol) of diethyl 9,10-dihydrophenanthrene-2,7-dicarboxylate were obtained as a by-product (selectivity of 0.2%).

## EXAMPLE 6

An autoclave was charged with 4.23 grams (12.5 mmol) of 2,7-dibromo-9,10-dihydrophenanthrene, 3.03 grams (30 mmol) of ethyldimethylamine, 22.2 milligrams (0.125 mmol) of palladium chloride, 65.6 milligrams (0.25 mmol) of triphenylphosphine, 10 milliliters of ethanol and 20 milliliters of benzene. The reaction was carried out for 3 hours at a temperature of 150 °C under a carbon monoxide pressure of 50 $kg/cm^2$ as in Example 1. 64% of the charged 2,7-dibromo-9,10-dihydrophenanthrene reacted. 2.09 grams (6.3 mmol) of ethyl 7-bromo-9,10-dihydrophenanthrene-2-carboxylate were obtained as a chief product (selectivity of 78%). Besides, 0.52 gram (1.6 mmol) of diethyl 9,10-dihydrophenanthrene-2,7-dioarboxylate was obtained as a by-product (selectivity of 19%).

As can be seen from the foregoing, according to the present invention, the useful and novel compounds represented by the general formulae (I) and (II) can be obtained in a high yield and selectivity from inexpensive raw materials by simple reaction steps and therefore the present invention is extremely effective.

## Claims

1 . A 9, 10-dihydrophenanthrene-2,7-dicarboxylic acid diester represented by the general formula (I):

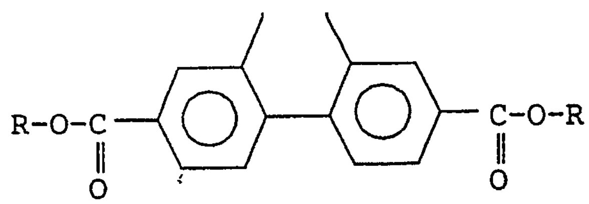

or a 7-bromo-9,10-dihydrophenanthrene-2-carboxylic acid ester represented by the general formula (II):

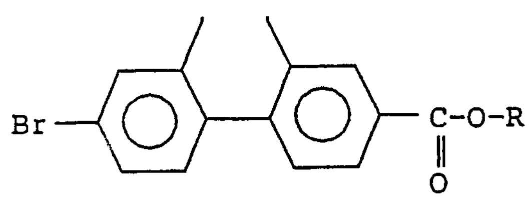

wherein each R represents a hydrocarbon group containing from 1 to 20 carbon atoms.

2. The compound according to claim 1 wherein R in the general formulae is selected from the group consisting of aliphatic, alicyclic and aromatic groups.

3. The compound according to claim 2 wherein R in the general formulae is selected from the group consisting of alkyl, cycloalkyl and aryl groups.

4. A process for selectively synthesizing the 9,10-dihydrophenanthrene-2,7-dicarboxylic acid diester and the 7-bromo-9,10-dihydrophenanthrene-2-carboxylic acid ester according to claim 1 which comprises the step of reacting a 2,7-dibromo-9,10-dihydrophenanthrene with carbon monoxide and an alcohol of the general formula (III) R-OH wherein R is a hydrocarbon group containing from 1 to 20 carbon atoms in the presence of a base and a palladium-phosphine catalyst.

5. The process according to claim 4 wherein the base is selected from the group consisting of aliphatic amines, aromatic amines, quaternary ammonium hydroxide, alkali metal alcoholates, alkali metal hydroxides, alkali metal carbonates, alkaline earth metal alcoholates, alkaline earth metal hydroxides and alkaline earth metal carbonates.

6. The process according to claim 4 wherein the base is used in an amount of from 0.01 to 10 molar equivalents, preferably from 0.3 to 5 molar equivalents based on the amount of bromine in the 2,7-dibromo-9,10-dihydrophenanthrene to be reacted.

7. The process according to claim 4 wherein palladium in the palladium-phosphine catalyst is derived from a compound selected from the group consisting of palladium chloride and palladium sulfate.

8. The process according to claim 4 wherein phosphine in the palladium-phosphine catalyst is selected

from the group consisting of aromatic phosphines, alkyl phosphines and diphosphines.

9. The process according to claim 4 wherein palladium is used in an amount of from 1/10 to 1/1000 molar equivalents, preferably from 1/20 to 1/200 molar equivalents based on 2,7-dibromo-9,10-dihydrophenanthrene to be reacted.

10. The process according to claim 4 wherein phosphine is used in an amount of from 0.5 to 30 molar equivalents, preferably from 0.5 to 30 molar equivalents based on palladium.

11. The process according to claim 4 wherein the alcohol is selected from the group consisting of aliphatic alcohols, alicyclic alcohols and aromatic alcohols.

12. The process according to claim 4 wherein the alcohol is used in an amount of at least one molar equivalent based on bromine in the 2,7-dibromo-9,10-dihydrophenanthrene to be reacted.

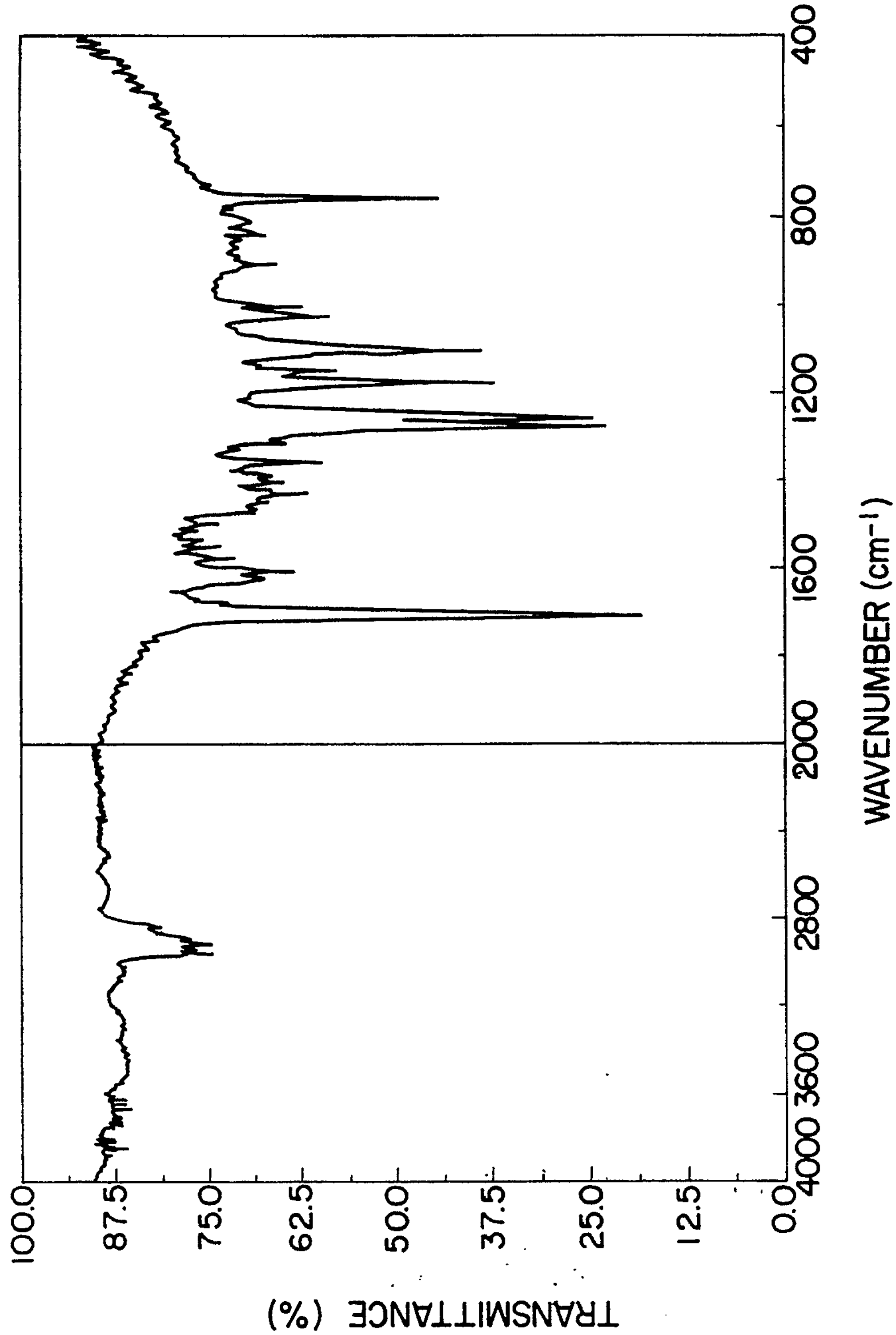
TRANSMITTANCE (%)
100.0
87.5
75.0
62.5
50.0
37.5
25.0
12.5
0.0
4000
3600
2800
2000
1600
1200
800
400
WAVENUMBER (cm$^{-1}$)

F I G. I

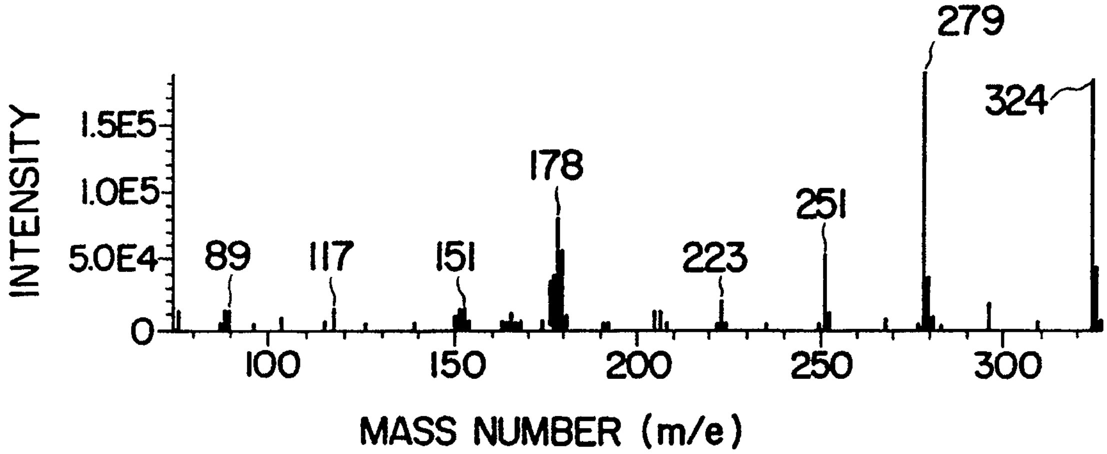
279
324
178
251
89
117
151
223
INTENSITY
1.5E5
1.0E5
5.0E4
0
100
150
200
250
300
MASS NUMBER (m/e)

FIG. 2

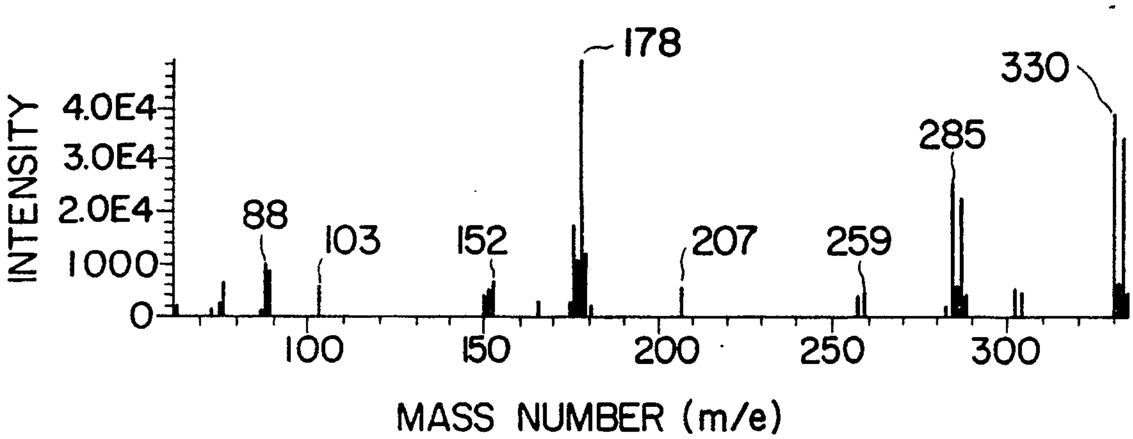
178
330
285
88
103
152
207
259
INTENSITY
4.0E4
3.0E4
2.0E4
1000
0
100
150
200
250
300
MASS NUMBER (m/e)

FIG. 4

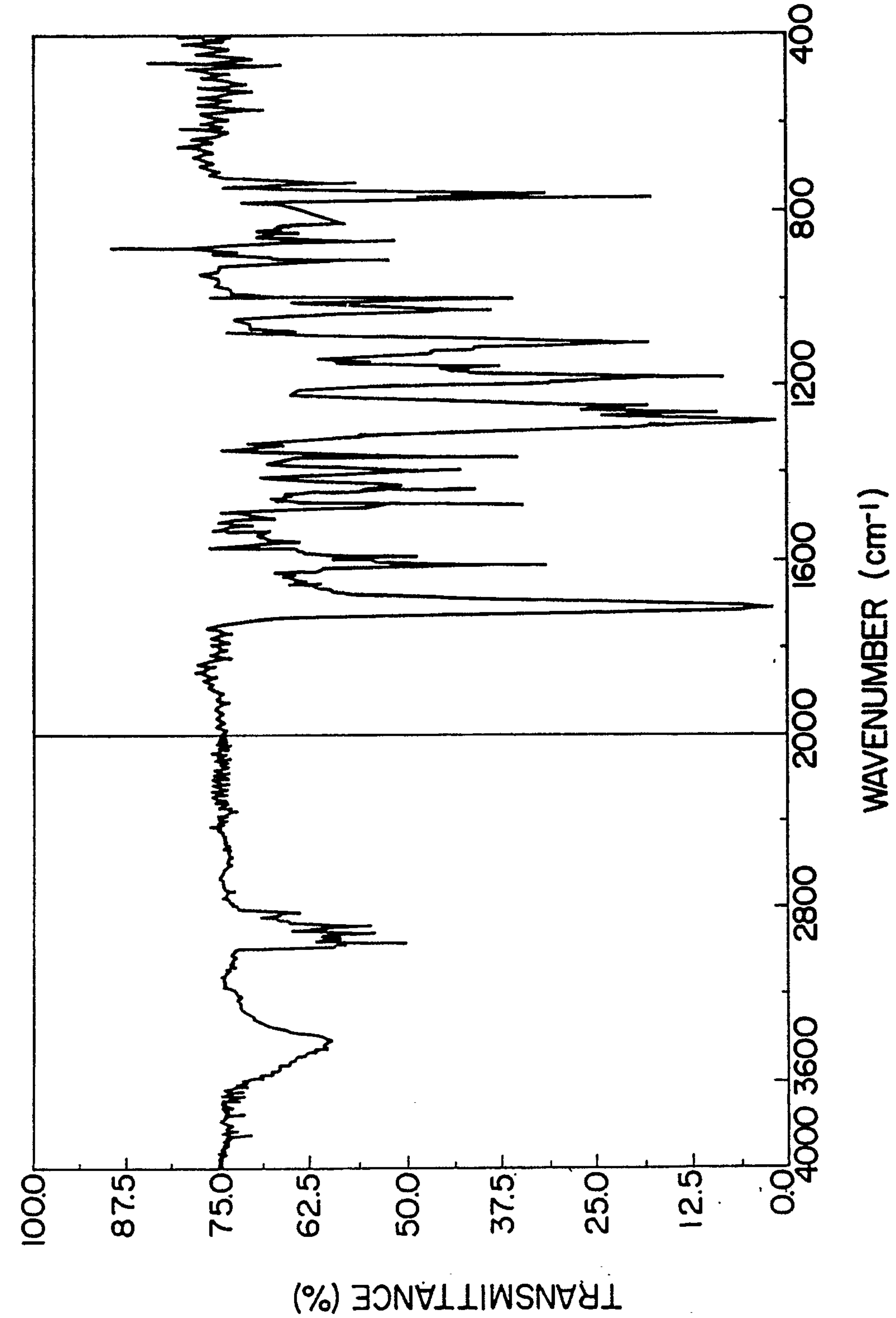
TRANSMITTANCE (%)
100.0
87.5
75.0
62.5
50.0
37.5
25.0
12.5
0.0
4000
3600
2800
2000
1600
1200
800
400
WAVENUMBER (cm⁻¹)

F I G. 3

European Patent Office

# EUROPEAN SEARCH REPORT

Application number

EP 90304881.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.⁵) |
|---|---|---|---|
| X | CHEMICAL ABSTRACTS, vol. 72, no. 13, March 30, 1970, Columbus, Ohio, USA R. PAIONI et al. "Higher condensed ring systems.5. Macrocyclic compounds with bridged 9-10-dihydro-phenanthrene units" page 368, column 2, page 369, column 1, abstract-no. 66 693w & Helv. Chim. Acta 1970, 53(1), 141-54 + CHEMICAL ABSTRACTS, 8th Collective Index, 1967-1971, Columbus, Ohio, USA FORMULAS C15-C18 page 7023F, column 1, lines 4-6 from the bottom -- | 1-3 | C 07 C 69/76<br>C 07 C 67/36 |
| A | CHEMICAL ABSTRACTS, vol. 98, no. 19, May 9, 1983, Columbus, Ohio, USA S. RICHTER et al. "Liquid crystalline 7-n-alkyl-9,10-dihydrophenantrene-2-carboxylates" page 642, column 1, abstract-no. 170 786b & Z. Chem. 1983, 23(1), 21-2 -- | 1 | TECHNICAL FIELDS SEARCHED (Int. Cl.⁵)<br>C 07 C 67/00<br>C 07 C 69/00<br>C 07 C 63/00 |
| A | US - A - 3 988 358 (R.F. HECK) * Totality * ---- | 4-12 | |

The present search report has been drawn up for all claims

| Place of search | Date of completion of the search | Examiner |
|---|---|---|
| VIENNA | 03-08-1990 | HOFBAUER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82